(19) Europäisches Patentamt
European Patent Office
Office européen des brevets

(11) Publication number: **0 019 602**
**A1**

(12) **EUROPEAN PATENT APPLICATION**

(21) Application number: 80850066.4

(22) Date of filing: 29.04.80

(51) Int. Cl.³: **A 61 K 7/16**

(30) Priority: 03.05.79 SE 7903856

(43) Date of publication of application: 26.11.80 Bulletin 80/24

(84) Designated Contracting States: AT BE CH DE FR GB IT LI NL SE

(71) Applicant: DENTAL THERAPEUTICS AB
3, Ektorpsvägen
S-131 45 Nacka(SE)

(72) Inventor: Wahlstam, Hans
110 Dalkärrsleden
S-162 24 Vällingby(SE)

(74) Representative: Neihoff, Arne
AB STOCKHOLMS PATENTBYRA Zacco & Bruhn Box 3129
S-103 62 Stockholm(SE)

(54) **Tooth-paste.**

(57) Tooth-paste comprising a hydrocarbon free of halogen, an emulsifier, a tenside, a thickener and water, in which
the content of hydrocarbon is 1-10 % by weight
the content of emulsifier is 1-10 % by weight
the content of tenside is 1-5 % by weight, and
the content of thickener is 0.5-5 % by weight
and optionally moisture retaining agent in an amount of up to 30 % by weight and optionally other current additives to tooth-paste, such as abrasives and flavouring agents, the rest consisting of water and the content of hydrocarbon and emulsifier being of substantially the same order of magnitude and the hydrocarbon being present in solubilized and evenly distributed state in the tooth-paste in form of gel.

EP 0 019 602 A1

Croydon Printing Company Ltd.

# Tooth-paste

This invention relates to a new tooth-paste.

The tooth-paste is a new agent for cleaning teeth based on the principle that no strong abrasive is required, nor any brushing with a hard toothbrush. The plaque layer should instead be removed by means of dissolution.

All known teeth cleaning principles for individual use are based on the principle of toothbrushing, i.e. that the teeth must be brushed to be clean by means of a tooth cleaning agent containing rather strong abrasives, such as chalk, phosphate, bicarbonate etc. Usually the cleaning ability of these agents is totally related to the grinding ability of the abrasive. In course of time the use of strong abrasives combined with hard nylon brushes has been found to cause microscopic scratches in the dental enamel which, in its turn, increases the possibility of a layer of plaque being built up again as these scratches increase the possibility of fixation to the teeth surfaces. Moreover, damage to the necks of the teeth will arise due to wear, which results in hyper-sensibility. The method of brushing teeth is also a matter of dispute, and parts of the plaque layer and abrasive can be easily brushed down into gingival pockets if an erroneous technique is used, which causes minor infections and inflammations resulting, in its turn, in paradontosis with loosening teeth as a consequence.

Today's tooth-paste is much built on the fact that the individual should feel a "fresh" taste in his mouth. This has the negative effect that you will have the false feeling of being "clean" in your mouth. Therefore toothbrushing will be incomplete.

As a consequence of this many expert dentists and researchers are of the opinion that today's tooth-paste is of a dubious value. Most dentists desire a tooth-paste not causing abrasive damage but facilitating removal of plaque.

Now it has been found that plaque can be removed by means of this new tooth-paste, another "toothbrushing" method preferably also being used. The tooth-paste is characterized by a hydrocarbon free of halogen, an emulsifier, a tenside, a thickener and water, in which

the content of hydrocarbon is 1-10 % by weight
the content of emulsifier is 1-10 % by weight
the content of tenside is 1-5 % by weight, and
the content of thickener is 0.5-5 % by weight

and optionally moisture retaining agent in an amount of up to 30 % by weight and optionally other current additives to tooth-paste, such as abrasives and flavouring agents, the rest consisting of water and the content of hydrocarbon and emulsifier being of substantially the same order of magnitude and the hydrocarbon being present in solubilized and evenly distributed state in the tooth-paste in form of gel. The hydrocarbon free of halogen, i.e. non-halogenous hydrocarbon has preferably an isoprenoid structure and the best results have been achieved with monocyclic terpene, above all limonene. Of course the hydrocarbon free of halogen can contain minor amounts of other substances which do not influence its function without the scope of the invention therefore being exceeded. The emulsifier is preferably a nonionic emulsifier and the tenside is preferably amphoteric. Besides, small amounts of alkali fluoride, such as sodium fluoride, flavouring agents (aromes), preservatives etc. can be included in the

tooth-paste of the invention. Their amount is totally usually below 5 % by weight, conveniently below 2 % by weight and preferably about 1 % by weight.

Hydrocarbons with an isoprenoid structure are e.g. ocimene, myrcene, D-limonene, α-terpinene, β-terpinene, λ-terpinene, δ-carene, sabinene, α-pinene and camphene. The monocyclic terpenes and above all limonene are most suitable among these. Saturated hydrocarbons, such as alkanes, e.g. pentanes, octanes, decanes, can also be used.

The tooth-paste according to the invention is rubbed onto the teeth surfaces and between the teeth without addition of water. Tooth-paste can be applied to the surfaces between the teeth by means of tooth-picks and/or by pressing the liquid in the mouth between the teeth. Accessible teeth surfaces are then rubbed again with a brush, after which the mouth is rinsed a few times with hot water. The effect of the tooth-paste can be explained by the fact - without this explanation being binding for the invention - that fats and lipides included in the plaque are dissolved and absorbed in the halogen-free hydrocarbon. The plaque will not be rubbed about on the teeth surfaces, which is done at cleaning according to known principles. It is known that plaque consists of lipides, polysaccharides and proteins and that these lipides are a prerequisite of calcification of plaque (tartar). Therefore it is important that these lipides are effectively removed from the tooth surface.

Addition of a very weak abrasive to the present tooth-paste accelerates plaque removal as well as rubbing with a soft toothbrush. It seems as if the droplets of halogen-free hydrocarbon in the tooth-paste will be transformed into larger drops, which

are more capable of dissolving the lipide in the plaque. As small an amount of water as possible should be present at cleaning. At a substantial plaque layer you can leave the tooth-paste a little longer (1-2 min.) on the teeth surfaces before the mouth is rinsed with lukewarm water. The present tooth-paste can be diluted with water without the lipides included therein migrating from the halogen-free hydrocarbon. In this way they can be carried away with the tooth cleaning agent.

The other parts of the plaque layer such as proteins and polysaccharides are broken up by the cleaning agent. The content of tensides is then likely to accelerate said breaking up. Proteins and polysaccharides included in the plaque layer then migrate into the tooth-paste and are carried away with this in its water phase when the mouth is rinsed.

In principle any hydrocarbon free of halogen might be used in the present tooth-paste. However, it is preferably a liquid at usual temperature and body temperature. Moreover, it should of course be nontoxic and preferably such hydrocarbons can be used as are allowed in foodstuffs. It has been found that monocyclic terpenes provide the best result both in respect of plaque removal and tolerance. As monocyclic terpenes in a useful non-purified form citrus oils (lemon oil) may be mentioned and as an example of clean monocyclic terpene limonene.

As emulsifiers nonionic surfactants can be used, which should preferably be completely soluble in the used fat dissolving substance. These should also be permitted for use in foodstuffs. As examples polyoxyethylene sorbitan monostearate ("TWEEN 60®"), polyoxyethylene sorbitan monooleate ("TWEEN 80®"), further polyoxyethylene sorbitan monopalmitate, monolaurate and myristate, sorbitan monostearate, monooleate and monopalmitate can be mentioned. Of these compounds the corresponding di- and tri-mers can also be used as well as such as are mixed with fatty acid groups. Other useful emulsifiers are glycerol mono-, di- and tri-fatty acid esters. Polyoxyethylene stearyl ether as well as the corresponding lauroyl, myristoyl, palmitoyl and oleoyl homologues are also useful. Polyoxyethylene fatty alcohols of the same homologue series are also useful.

Most suitable as tenside is a so-called balanced, amphoteric compound; preferably imidazoline derivatives are used here.

The ampholytic (amphoteric) tenside can preferably be a tenside in the following groups of tensides:

1. 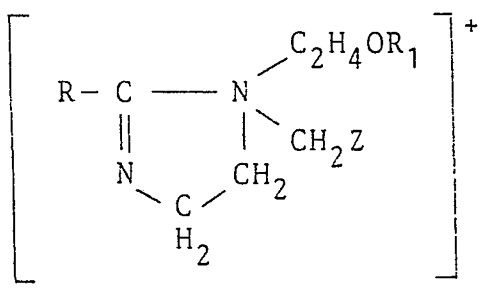 $OH^-$

wherein R is the acyl radical of a fatty acid with, on an average, 11-19 carbon atoms, conveniently 13-17 carbon atoms, preferably 14-16 carbon atoms, $R_1$ is $CH_2 \cdot COOM$ or M, Z is $-COOM$, $-CH_2 \cdot COOM$ or $-\underset{OH}{CH}-CH_2SO_3M$ and M is an alkaline metal, H or the radical of an organic base, preferably an amine, which can be a heterocyclic amine or a triloweralkyl amine or a triloweralkanol amine, e.g. triethyl amine or triethanol amine.

2. $$R_2 - NH - (CH_2)_x - \overset{CH_3}{\underset{CH_3}{N^+}} - CH_2 - COO^-$$

wherein $R_2$ is the acyl radical of a fatty acid with, on an average, 12-18 carbon atoms, and x is an integar of the value 1-4, preferably 2-3.

3. 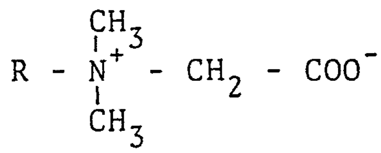

wherein R is an alkyl group, conveniently with 12-18 carbon atoms, preferably a mixture of 12-18 carbon atoms.

4. $R_3 - NH - (CH_2)_x - NH - CH_2 - COO^-$

wherein $R_3$ is an alkyl group with, on an average, 8-18 carbon atoms, conveniently 10-14 carbon atoms, preferably 12 carbon atoms, and x is an integer of the value 1-4, preferably 2-3.

To sum up, these different groups of tensides 2-4 can be written as

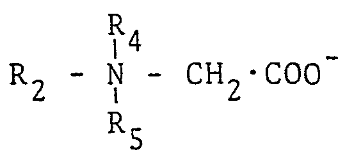

wherein $R_2$ is an alkyl group, conveniently with 12-18 carbon atoms, preferably a mixture of 12-18 carbon atoms or an alkylaminoethyl or alkylaminopropyl group with 8-18 carbon atoms, conveniently 10-14, preferably 12 carbon atoms (also a mixture with 8-18 carbon atoms is possible, or an acylaminoethyl or an acylaminopropyl group, where the acyl group has 12-18 carbon atoms on an average, preferably a mixture of 12-18 carbon atoms, and where $R_4$ and $R_5$ either both are metyl groups or where $R_4$ is H and $R_2$ an electron pair.

However, it is also possible to use anionic tensides, e.g. sodium lauryl alcohol sulfate and sulfonate and their homologues with respect to the

number of carbon atoms in the fatty alcohol. Also nonionic tensides can be concerned, the polyethoxylic chain having a varying length. Quaternary tensides are also useful.

The thickener consists preferably of carboxymethyl cellulose, a cellulose ether or a microcrystalline cellulose. Other useful agents are polyglycols, alginate and alginic acid propylene glycol ester, gelatine, starch and so-called "mucilage", e.g. carrageenin, sterculia gum and tragacanth.

Moreover, the tooth-paste contains preferably a water binding agent (moisture retaining), a so-called "humectant" which is a usual constituent in all toothpastes. A suitable addition is 5-30 % by weight of the tooth-paste, preferably 10-20 % by weight. Suitable moisture retaining agents are glycerol. However, e.g. propylene glycol and sorbitol and other polyalcohols are also useful.

Abrasives optionally used should also be mild and a suitable abrasive is a fine granular silicon dioxide, e.g. that sold under the name of "CAB o SIL". The tooth-paste can also contain a fluoride, such as sodium fluoride, and the usual flavouring agents and preservatives. The thickener is added in such an amount that a suitable consistency is achieved. The fluoride can be used up to the maximally permitted amount.

The invention is described more closely in the following examples.

Example 1

| | |
|---|---|
| Orange terpenes | 5.0 g |
| "TWEEN 60" | 5.0 g |
| "CAB o SIL" | 2.5 g |
| Amphoteric-2 of 38 % | 5.0 g |
| CMC | 1.0 g |
| Flavouring agents | suitable amount |
| Preservatives | suitable amount |
| Glycerol | 10.0 g |
| Sodium fluoride | 0.25 g |
| Water | up to 100.0 g |

Example 2

| | |
|---|---|
| Limonene | 5.0 g |
| "TWEEN 80" | 5.0 g |
| Amphoteric-2 | 5.0 g |
| CMC | 2.5 g |
| Glycerol | 10.0 g |
| Sodium fluoride | 0.25 g |
| Flavouring agents | sufficient amount |
| Preservatives | sufficient amount |
| Water | up to 100.0 g |

Claims

1. Tooth-paste, characterized by a hydrocarbon free of halogen, an emulsifier, a tenside, a thickener and water, in which
the content of hydrocarbon is 1-10 % by weight
the content of emulsifier is 1-10 % by weight
the content of tenside is 1-5 % by weight, and
the content of thickener is 0.5-5 % by weight
and optionally moisture retaining agent in an amount of up to 30 % by weight and optionally other current additives to tooth-paste, such as abrasives and flavouring agents, the rest consisting of water and the content of hydrocarbon and emulsifier being of substantially the same order of magnitude and the hydrocarbon being present in solubilized and evenly distributed state in the tooth-paste in form of gel.

2. Tooth-paste as claimed in claim 1, characterized in that the hydrocarbon free of halogen is a monocyclic terpene.

3. Tooth-paste as claimed in claim 2, characterized in that the hydrocarbon free of halogen is a citrus oil.

4. Tooth-paste as claimed in claim 2, characterized in that the hydrocarbon free of halogen is limonene.

5. Tooth-paste as claimed in any one of claims 1-4, characterized in that the emulsifier is nonionic.

6. Tooth-paste as claimed in any one of claims 1-5, characterized in that the tenside is amphoteric.

7. Tooth-paste as claimed in any one of claims 1-6, characterized in that it comprises, in parts by weight, 3-8 parts of limonene, 3-8 parts of emulsifier, 2-7 parts of tenside, 2-3 parts of abrasive, 5-15 parts of moisture retaining agent,

1-3 parts of thickener and 45-95 parts of water, this composition being 100 parts, also small amounts of fluoride, flavouring agents and preservatives in an amount of maximum 5 parts totally being included in the tooth-paste.

0019602

Application number

European Patent Office

# EUROPEAN SEARCH REPORT

EP 80 85 0066

## DOCUMENTS CONSIDERED TO BE RELEVANT

| Category | Citation of document with indication, where appropriate, of relevant passages | Relevant to claim |
|---|---|---|
| | US - A - 3 705 940 (H. KIRCHGASSNER) * The whole document * | 1-7 |
| | FR - A - 2 194 411 (UNILEVER N.V.) * Page 1, lines 1-4; page 1, line 36 - page 3, line 36; example 2; claims 1,8 * | 1-7 |
| | US - A - 3 876 759 (COLGATE PALMOLIVE) * Column 1, lines 4-29; column 1, lines 52-58; column 2, line 17 - column 3, line 11 * | 1-5 |
| | HUGO JANISTYN: "Handbuch der Kosmetika und Riechstoffe", vol. 1, Edition 2, Hüthig, 1969 Heidelberg, DE. pages 740-741 "Paraffinöle" | 1 |
| | FR - A - 2 201 865 (COLGATE PALMOLIVE) * Page 1, line 1 - page 2, line 29; page 4, line 20 - page 7, line 16; page 8, line 26 - page 9, line 30 * | 1-7 |
| A | US - A - 3 574 824 (J. ECHEANDIA) * Column 1, lines 13-69; column 2, line 46 - column 6, line 7; column 7, lines 14-20; examples 1-5 * | 1-7 |

./.

CLASSIFICATION OF THE APPLICATION (Int. Cl. 3)

A 61 K 7/16

TECHNICAL FIELDS SEARCHED (Int.Cl. 3)

A 61 K 7/16

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 20-08-1980 | CONTET |

EPO Form 1503.1 06.78

European Patent Office

# EUROPEAN SEARCH REPORT

Application number EP 80 85 0066



| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | FR - M - 7536 (AKTIEBOLAGET BOFORS)<br>* Page 1, left-hand column, lines 1-4; page 1, right-hand column, line 23 - page 2, left-hand column, line 5; examples 1-4 *<br>-- | 1,7 | |
| A | FR - A - 2 173 996 (DEUTSCHE GOLD- UND SILBER-SCHEIDEANSTALT)<br>* Page 1, line 1 - page 3, line 12; examples 1,2; page 5, lines 29-38 *<br>-- | 1-7 | TECHNICAL FIELDS SEARCHED (Int. Cl. 3) |
| A | FR - A - 778 232 (BEHR)<br>* The whole document *<br>---- | 1,7 | |